Europäisches Patentamt
European Patent Office
Office européen des brevets

(19)

(11) Numéro de publication: **0 156 785**
**A1**

(12)

# DEMANDE DE BREVET EUROPEEN

(21) Numéro de dépôt: 85870036.2

(22) Date de dépôt: 06.03.85

(51) Int. Cl.⁴: C 07 C 97/10
C 07 D 295/10, C 07 C 263/56
A 61 K 31/135, A 61 K 31/3-95

(30) Priorité: 13.03.84 LU 85246

(43) Date de publication de la demande:
02.10.85 Bulletin 85/40

(84) Etats contractants désignés:
AT BE CH DE FR GB IT LI NL SE

(71) Demandeur: S.A.R.L. GALEPHAR
Zone Industrielle A rue du Mont de Templemars
F-59139 Noelles les Seclin(FR)

(72) Inventeur: Cazin, Micheline
rue Destailleurs, 111
F-59045 Lille(FR)

(72) Inventeur: Lesieur, Isabelle
rue de Verdun, 20
F-59147 Gondecourt(FR)

(72) Inventeur: Lesieur, Daniel
rue de Verdun, 20
F-59147 Gondecourt(FR)

(72) Inventeur: Lespagnol, Charles
Avenue Pasteur, 115
F-59130 Lambersarb(FR)

(72) Inventeur: Delacourte, André
rue Gambetta, 75
F-59155 Faches Thumesnil(FR)

(72) Inventeur: Baudier, Philippe
Avenue Blücher, 12
B-1410 Waterloo(BE)

(72) Inventeur: Bianchi, André
Square d'Espagne, 31/12
F-59000 Lille(FR)

(74) Mandataire: Schmitz, Yvon et al,
Bureau Gevers S.A. 7, rue de Livourne Bte 1
B-1050 Bruxelles(BE)

(54) **2-(aminoalkyl)acrylophénones, leur préparation et leur utilisation.**

(57) 2-(aminoalkyl)acrylophénones de la formule:

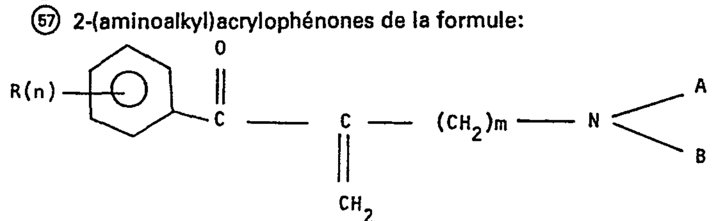

dans laquelle:

A et B désignent de l'hydrogène ou un radical alkyle inférieur ou phényle substitué ou non, ou avec l'atome d'azote auquel ils sont liés, un radical hétérocyclique;
les symboles R désignent de l'hydrogène, un halogène, un groupe alkyle inférieur substitué ou non, un acide carboxylique, sulfonique ou un ester de ces acides, un amide substitué ou non, un groupe nitro, hydroxy, éther d'alkyle inférieur, un groupe phényle ou phénoxy substitué ou non ou un hétérocyle, n vaut de 1 à 3 et m de 1 à 5, leur préparation et leur utilisation.

Comme médicaments et spécialement comme hypolipémiant et hypocholestérolémiant.

EP 0 156 785 A1

La présente invention concerne de nouveaux dérivés de la 2-acrylophénone, leurs sels pharmaceutiquement acceptables, leur procédé de préparation ainsi que les médicaments qui les contiennent.

Les composés de la présente invention répondent à la formule générale (I):

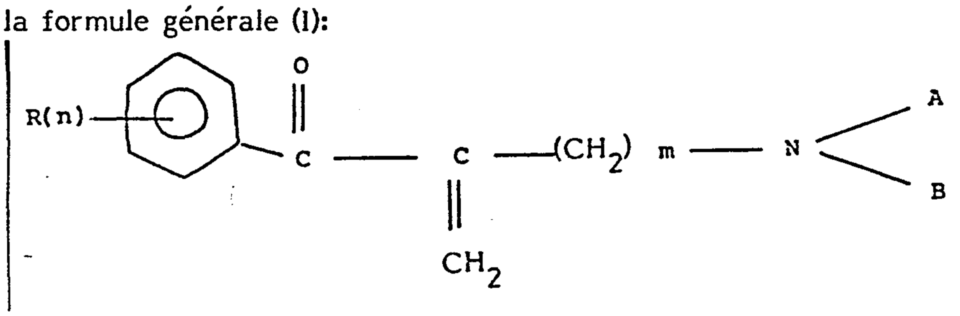

(I)

dans laquelle :

A et B peuvent être identiques ou non et désignent un atome d'hydrogène, un radical alkyle inférieur, un radical phényle substitué ou non, ou avec l'atome d'azote auquel ils sont liés, un radical hétérocyclique, tel qu'un radical morpholino, pipérazinyle, 4-méthylpipérazinyle, pipéridino, pyrrolidino substitué ou non;

les symboles R, qui peuvent être identiques ou différents, désignent de l'hydrogène, un halogène, un groupe alkyle inférieur, un groupe alkyle inférieur substitué par 1 à 3 atomes d'halogène identiques ou différents, un acide carboxylique, sulfonique ou un ester de ces acides, un amide substitué ou non, un groupe nitro, hydroxy, éther d'alkyle inférieur tel que méthoxy, éthoxy, n- ou isopropyloxy, un groupe phényle ou phénoxy substitué ou non, un hétérocycle, tel que la 3-méthyl oxazole-2-one;

n est le nombre 1, 2, 3; et
m est le nombre 1, 2, 3, 4, 5.

Les 2-(aminoalkyl)acrylophénones de la présente invention peuvent se présenter soit sous forme de base soit sous forme de sels pharmaceutiquement acceptables, tels que chlorhydrates, bromhydrates, sulfates ou des sels d'acides organiques.

Les acrylophénones de la présente invention sont dotées d'activités thérapeutiques remarquables et possèdent d'excellentes propriétés anticancéreuses, antiparasitaires, antimicrobiennes, antifongiques, hypolipémiantes, hypocholestérolémiantes, diurétiques, antiseptiques urinaires, antiinflammatoires, antiagrégantes plaquettaires et analgésiques.

On a décrit ci-après deux procédés de préparation des 2-(aminoalkyl)acrylophénones de la présente invention.

<u>Procédé I</u>: Permet l'obtention des 2-(aminométhyl)acrylophénones(m = 1). Le procédé consiste à mettre en réaction une amine du type (II) sous forme de base ou de chlorhydrate, le formaldéhyde et une acétophénone (III) convenablement substituée en milieu d'acide acétique. Après chauffage au reflux pendant quelques heures, on évapore à sec le milieu réactionnel et la 2-(aminométhyl)acrylophénone est recristallisée dans un solvant approprié (schéma I).

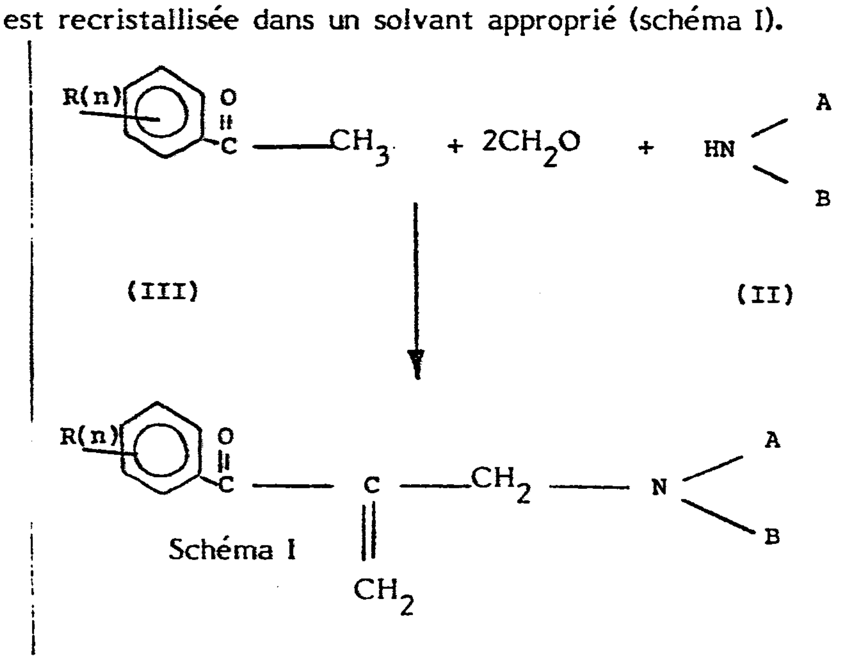

(III) (II)

Schéma I

<u>Procédé II:</u> Permet l'obtention des 2-(aminoalkyl)acrylophénones de formule générale (I)
Le procédé consiste à mettre en réaction une aminoalkylphénone (IV) avec du formaldéhyde en milieu d'acide acétique. Après avoir porté le mélange réactionnel à reflux pendant quelques heures, on évapore le solvant sous vide et la 2-(aminoalkyl)acrylophénone est recristallisée dans un solvant approprié (schéma II).

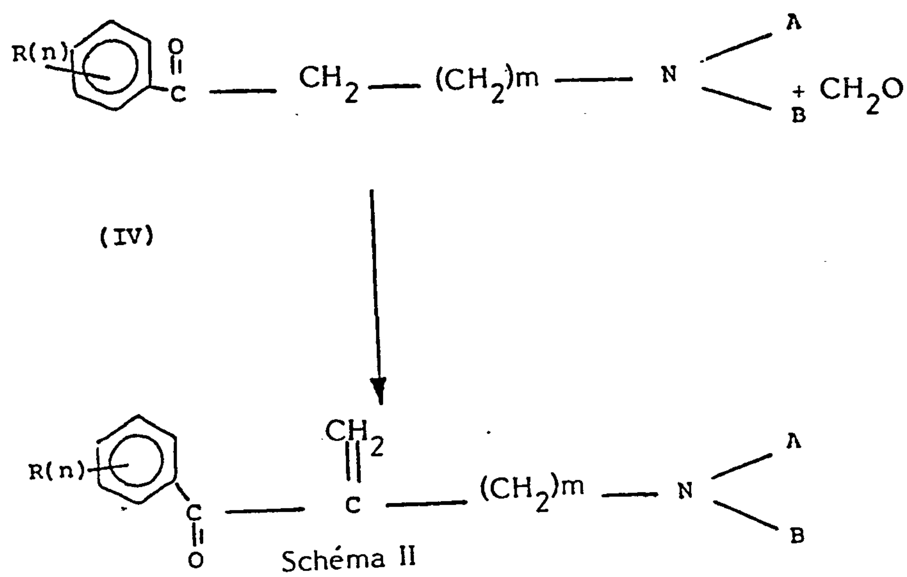

(IV)

Schéma II

Les produits de cette invention peuvent être administrés par voie parentérale, topique ou per os et on peut les mélanger comme on le désire avec des diluants, des solvants, des agents de mise en suspension, des excipients, des adhésifs, des matières colorantes ou aromatisantes pour la préparation de formes posologiques appropriées.

Les exemples ci-après sont destinés à illustrer la préparation des composés de la présente invention et ne peuvent en aucun cas la limiter.

<u>Exemple 1 :</u>Dichlorhydrate de 2-(4-méthyl-1-pipérazinylméthyl)acrylophénone.

3 g (0,1 M) de trioxyméthylène et 8,65 g (0,05 M) de dichlorhydrate de N-méthylpipérazine sont ajoutés à 6 g d'acétophénone dans 20 ml d'acide acétique. Le mélange est chauffé à reflux pendant 3 h. Le solvant est éliminé et le résidu jaune huileux est dissous dans 50 ml d'éthanol. Après élimimation du solvant, le résidu cristallise dans du méthanol. On a obtenu 9,5 g du produit cité en rubrique ayant pour point de fusion : 231-233°C et dont les caractéristiques sont : poudre cristalline blanche, très soluble dans l'eau, peu soluble dans l'alcool et pratiquement insoluble dans l'acétone.

Analyse élémentaire :

| | C% | H% | N% | O% | Cl% |
|---|---|---|---|---|---|
| calculé : | 56,79 | 6,99 | 8,83 | 5,04 | 22,35 |
| trouvé : | 56,67 | 6,92 | 8,68 | 5,28 | 22,04 |

Exemple 2 :Dichlorhydrate de 2-(4-méthyl-1-pipérazinylméthyl)acrylophone.

En procédant de la même manière que dans l'exemple 1, mais en partant de 0,05 mole (15,26 g) de chlorhydrate de 3-(N-méthylpipérazinyl)-propiophénone et de 0,05 mole (1,5 g) de formaldéhyde sous forme de trioxyméthylène, on a obtenu 7,9 g de produit cité en rubrique ayant pour point de fusion 232°C et en tous points identique au produit de l'exemple 1.

En procédant comme dans l'exemple 1 ou 2, les produits suivants ont été obtenus :

- dichlorhydrate de 2-(4-méthyl-pipérazinylméthyl)-acrylophénone solvant de recristallisation : méthanol, point de fusion : 231-233°C
- chlorhydrate de 2-(1-morpholinométhyl)-acrylophénone solvant de recristallisation : isopropanol, point de fusion: 182-184°C
- chlorhydrate de 2-(N,N-diméthylaminométhyl)-acrylophénone solvant de recristallisation : acétone, point de fusion: 158-160°C

- dichlorhydrate de 2-(4-méthyl-1-pipérazinylméthyl)-p-chloroacrylophénone
solvant de recristallisation : isopropanol, point de fusion: 215-220°C.
- chlorhydrate de 2-(1-morpholinométhyl)-p-chloroacrylophénone
solvant de recristallisation : acétonitrile, point de fusion: 201-207°C
- chlorhydrate de 2-(N,N-diméthylaminométhyl)-p-chloroacrylophénone
solvant de recristallisation : dioxane, point de fusion: 176-179°C
- dichlorhydrate de 2-(4-méthyl-1-pipérazinylméthyl)-p-méthoxyacrylophénone
solvant de recristallisation : éthanol, point de fusion: 214-224°C.
- chlorhydrate de 2-(1-morpholinométhyl)-p-méthoxyacrylophénone
solvant de recristallisation : isopropanol, point de fusion: 180-181°C.
- chlorhydrate de 2-(N,N-diméthylaminométhyl)-p-méthoxyacrylophénone
solvant de recristallisation : dioxane, point de fusion: 149-153°C
- chlorhydrate de 2-(1-morpholinométhyl)-3,4,5-triméthoxyacrylophénone
solvant de recristallisation : isopropanol, point de fusion: 173-177°C
- chlorhydrate de 2-(1-morpholinométhyl)-2,3,4-triméthoxyacrylophénone
solvant de recristallisation : isopropanol, point de fusion: 170-175°C
- chlorhydrate de 6-(3-diméthylamino-2-méthylène-propionyl)-3-méthyl-benzoxazolinone
solvant de recristallisation : éthanol, point de fusion: 208°C
- chlorhydrate de 6-(3-diéthylamino-2-méthylène- propionyl)-3-méthyl- benzoxazolinone
solvant de recristallisation : isopropanol, point de fusion: 178°C
- chlorhydrate de 6-(3-morpholino-2-méthylène-propionyl)-3-méthylbenzoxazolinone
solvant de recristallisation : éthanol, point de fusion: 226°C
- chlorhydrate de 2-(1-morpholinométhyl)-3,4-dichloroacrylophénone
solvant de recristallisation : isopropanol, point de fusion: 171-174°C

-chlorhydrate de 2-(1-morpholinométhyl)-2,4-dichloroacrylophénone solvant de recristallisatin : isopropanol, point de fusion: 194-197°C
- chlorhydrate de 2-(1-morpholinométhyl)-p-nitroacrylophénone solvant de recristallisation : acétate d'éthyle, point de fusion: 200-205°C.

Les dérivés de la présente invention présentent en outre des activités hypolipémiantes et hypocholestérolémiantes remarquables et peuvent donc trouver des applications en médecine humaine.
Cette propriété a été mise en évidence chez le rat au moyen de la méthode utilisant le Triton WR 1339 comme inducteur d'hyperlipémie.

Quatre heures après l'injection I.V. aux rats de 175 mg de Triton WR 1339 par kg de poids corporel, on leur injecte par voie intrapéritonéale soit du sérum physiologique (témoins) soit la substance à étudier. Les rats sont sacrifiés 24 heures après l'injection du Triton WR 1339 et les plasmas recueillis pour les dosages (résultats : Tableau I) des triglycérides et du cholestérol.

TABLEAU I.

Diminution de la l'hypertriglycéridémie et de l'hypercholestérolémie, provoquée chez le rat par le Triton WR 1339, au moyen des dérivés de la présente invention.

| Triton WR 1339 | Traitement ultérieur | Dose de traitement ultérieur(mg/kg) | Diminution(%)sous l'influence du traitement | |
|---|---|---|---|---|
| | | | triglycéridémie | cholestérolémie |
| 175 mg/kg (IV) | Néant | 0 | 0 | 0 |
| 175 mg/kg (IV) | dichlorhydrate de 2(4-méthyl-1-pipérazinylméthyl)acrylophénone | 4 (IP) | 47 | 57,5 |
| 175 mg/kg (IV) | chlorhydrate de 2(1-morpholinométhyl) acrylophénone | 4 (IP) | 44,5 | 51,0 |
| 175 mg/kg (IV) | chlorhydrate de 2(N,N-diméthylaminométhyl)acrylophénone | 2 (IP) | 36,5 | 48 |

Ces résultats font apparaître une diminution de la triglycéridémie de 36,5% à 47% et de la cholestérolémie de 48% à 57,5%

Les propriétés antiagrégantes plaquettaires des acrylophénones de la présente invention ont été mises en évidence au moyen d'adénosine triphosphate choisi comme agent inducteur de l'agrégation plaquettaire.

Les doses efficaces réduisant de 50% l'agrégation des plaquettes (D.E.50) ont été déterminées (Tableau II). Ces résultats montrent que les substances agissent à des doses de l'ordre micromolaire.

Tableau II

Activité antiagrégante plaquettaire (D.E.50)

| Acrylophénone | D.E.50mole/L |
|---|---|
| dichlorhydrate de 2(4-méthyl-1-pipérazinylméthyl) acrylophénone | 1,323.10-6 |
| chlorhydrate de 2(1-morpholinométhyl)acrylophénone | 1,939.10-6 |
| chlorhydrate de 2(N,N-diméthylaminométhyl) acrylophénone | 1,130.10-6 |

6. La 2-(N,N-diméthylaminométhyl)-p-chloroacrylophénone.

7. La 2-(4-méthyl-1-pipérazinylméthyl)-p-méthoxyacrylophénone.

8. La 2-(1-morpholinométhyl)-p-méthoxyacrylophénone.

9. La 2-(N,N-diméthylaminométhyl)-p-méthoxyacrylophénone.

10. La 2-(1-morpholinométhyl)-3,4,5-triméthoxyacrylophénone.

11. La 2-(1-morpholinométhyl)-2,3,4-triméthoxyacrylophénone.

12. La 2-(4-méthyl-1-pipérazinylméthyl)acrylophénone.

13. Le chlorhydrate de 6-(3-diméthylamino-2-méthylène- propionyl)-3-méthyl-benzoxazolinone.

14. Le chlorhydrate de 6-(3-diéthylamino-2-méthylène- propionyl)-3-méthyl-benzoxazolinone.

15. Le chlorhydrate de 6-(3-morpholino-2-méthylène-propionyl)-3-méthyl-benzoxazolinone.

16. Le chlorhydrate de 2-(1-morpholinométhyl)-3,4-dichloroacrylophénone.

17. Le chlorhydrate de 2-(1-morpholinométhyl)-2,4-dichloroacrylophénone.

18. Le chlorhydrate de 2-(1-morpholinométhyl)-p-nitroacrylophénone.

19. Procédé de préparation des 2-acrylophénones suivant l'une quelconque des revendications 1 à 12 et 16 à 18, caractérisé en ce que l'on fait réagir une amine de formule A-NH-B sous forme de base ou de sel avec une acétophénone de formule (II):

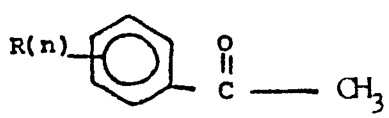

(II)

## REVENDICATIONS.

1. A titre de produits industriels nouveaux, les 2-acrylophénones répondant à la formule (I) :

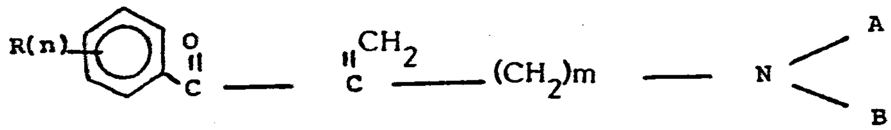

(I)

dans laquelle :

- A et B peuvent être identiques ou non et désignent un atome d'hydrogène, un radical alkyle inférieur, un radical phényle substitué ou non, ou avec l'atome d'azote auquel ils sont liés, un radical hétérocyclique, tel qu'un radical morpholino, pipérazinyle, 4-méthylpipérazinyle, pipéridino, pyrrolidino substitué ou non;
- les symboles R, qui peuvent être identiques ou différents, désignent de l'hydrogène, un halogène, un groupe alkyle inférieur, un groupe alkyle inférieur substitué par 1 à 3 atomes d'halogène identiques ou différents, un acide carboxylique, sulfonique ou un ester de ces acides, un amide substitué ou non, un groupe nitro, hydroxy, éther d'alkyle inférieur, tel que méthoxy, éthoxy, n- ou isopropyloxy , un groupe phényle ou phénoxy substitué ou non, un hétérocycle, tel que la 3-méthyl oxazole -2-one;

n est le nombre 1, 2, 3; et

m est le nombre 1, 2, 3, 4, 5, et les sels non toxiques, pharmaceutiquement acceptables de ces composés.

2. La 2- (1-morpholinométhyl)-acrylophénone.

3. La 2-(N,N-diméthylaminométhyl)-acrylophénone.

4. La 2- (4-méthyl-1-pipérazinylméthyl)-p-chloroacrylophénone.

5. La 2-(1-morpholinométhyl)p -chloroacrylophénone.

en présence de formaldéhyde en milieu d'acide acétique, les symboles A, B, R et n étant tels que définis ci-dessus.

2. Procédé de préparation de 2-acrylophénones répondant à la formule (I) :

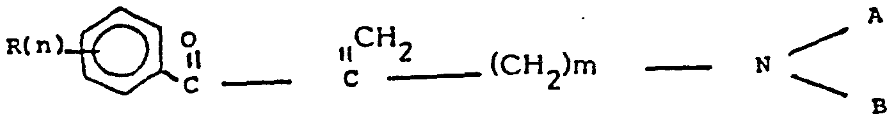

(I)

dans laquelle :

- A et B peuvent être identiques ou non et désignent un atome d'hydrogène, un radical alkyle inférieur, un radical phényle substitué ou non, ou avec l'atome d'azote auquel ils sont liés, un radical hétérocyclique, tel qu'un radical morpholino, pipérazinyle, 4-méthylpipérazinyle, pipéridino, pyrrolidino substitué ou non;

- les symboles R, qui peuvent être identiques ou différents, désignent de l'hydrogène, un halogène, un groupe alkyle inférieur, un groupe alkyle inférieur substitué par 1 à 3 atomes d'halogène identiques ou différents, un acide carboxylique, sulfonique ou un ester de ces acides, un amide substitué ou non, un groupe nitro, hydroxy, éther d'alkyle inférieur, tel que méthoxy, éthoxy, n- ou isopropyloxy, un groupe phényle ou phénoxy substitué ou non, un hétérocycle, tel que la 3-méthyl oxazole-2-one;

n est le nombre 1, 2, 3; et

m est le nombre 1, 2, 3, 4, 5, et les sels non toxiques, pharmaceutiquement acceptables de ces composés,

caractérisé en ce que l'on fait réagir du formaldéhyde avec une aminoalkylphénone de formule (III):

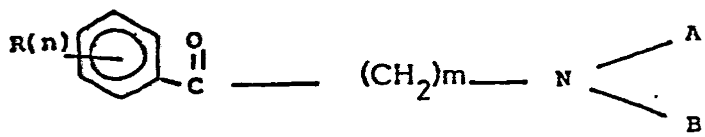

(III)

sous forme de base ou de sel en milieu d'acide acétique, les symboles A, B, R, n et m étant tels que définis ci-dessus.

3. Procédé suivant l'une ou l'autre des revendications 1 et 2, caractérisé en ce que l'acrylophénone est la 2-(1-morpholinométhyl)-acrylophénone.

4. Procédé suivant l'une ou l'autre des revendications 1 et 2, caractérisé en ce que l'acrylophénone est la 2-(N,N-diméthylaminométhyl)-acrylophénone.

5. Procédé suivant l'une ou l'autre des revendications 1 et 2, caractérisé en ce que l'acrylophénone est la 2-(4-méthyl-1-pipérazinylméthyl)-p-chloroacrylophénone.

6. Procédé suivant l'une ou l'autre des revendications 1 et 2, caractérisé en ce que l'acrylophénone est la 2-(1-morpholinométhyl)-p-chloroacrylophénone.

7. Procédé suivant l'une ou l'autre des revendications 1 et 2, caractérisé en ce que l'acrylophénone est la 2-(N,N-diméthylaminométhyl)-p-chloroacrylophénone.

8. Procédé suivant l'une ou l'autre des revendications 1 et 2, caractérisé en ce que l'acrylophénone est la 2-(4-méthyl-1-pipérazinylméthyl)-p-méthoxyacrylophénone.

9. Procédé suivant l'une ou l'autre des revendications 1 et 2, caractérisé en ce que l'acrylophénone est la 2-(1-morpholinométhyl)-p-méthoxyacrylophénone.

10. Procédé suivant l'une ou l'autre des revendications 1 et 2, caractérisé en ce que l'acrylophénone est la 2-(N,N-diméthylaminométhyl)-p-méthoxyacrylophénone.

11. Procédé suivant l'une ou l'autre des revendications 1 et 2, caractérisé en ce que l'acrylophénone est la 2-(1-morpholinométhyl)-3,4,5-triméthoxyacrylophénone.

REVENDICATIONS. (Autriche)

1. Procédé de préparation de 2-acrylophénones répondant à la formule (I)

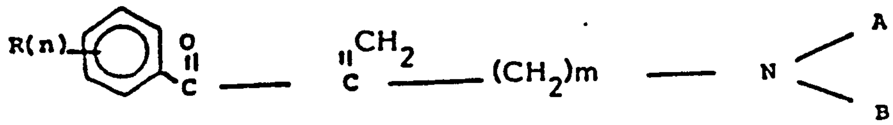

(I)

dans laquelle :

- A et B peuvent être identiques ou non et désignent un atome d'hydrogène, un radical alkyle inférieur, un radical phényle substitué ou non, ou avec l'atome d'azote auquel ils sont liés, un radical hétérocyclique, tel qu'un radical morpholino, pipérazinyle, 4-méthylpipérazinyle, pipéridino, pyrrolidino substitué ou non;

- les symboles R, qui peuvent être identiques ou différents, désignent de l'hydrogène, un halogène, un groupe alkyle inférieur, un groupe alkyle inférieur substitué par 1 à 3 atomes d'halogène identiques ou différents, un acide carboxylique, sulfonique ou un ester de ces acides, un amide substitué ou non, un groupe nitro, hydroxy, éther d'alkyle inférieur, tel que méthoxy, éthoxy, n- ou isopropyloxy , un groupe phényle ou phénoxy substitué ou non, un hétérocycle, tel que la 3-méthyl oxazole-2-one;

n est le nombre 1, 2, 3; et

m est le nombre 1, 2, 3, 4, 5, et les sels non toxiques, pharmaceutiquement acceptables de ces composés, caractérisé en ce que l'on fait réagir une amine de formule A-NH-B sous forme de base ou de sel avec une acétophénone de formule (II) :

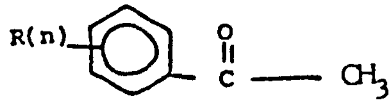

(II)

en présence de formaldéhyde en milieu d'acide acétique, les valeurs des symboles A, B, R, n étant telles que définies dans la revendication 1.

20. Procédé de préparation des 2-acrylophénones suivant l'une quelconque des revendications 1 à 18, caractérisé en ce que l'on fait réagir du formaldéhyde avec une aminoalkylphénone de formule(III):

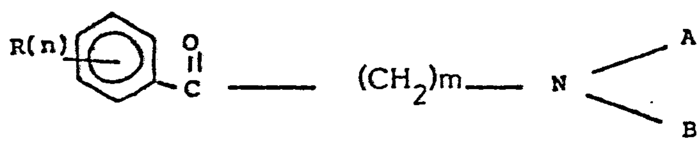 (III)

sous forme de base ou de sel en milieu d'acide acétique, les valeurs de A, B, R, n, m étant telles que définies dans la revendication 1.

21. Composition pharmaceutique comprenant, comme produit actif, au moins une acrylophénone suivant l'une quelconque des revendications 1 à 18, associée à au moins un excipient approprié et/ou au moins un autre agent thérapeutique

12. Procédé suivant l'une ou l'autre des revendications 1 et 2, caractérisé en ce que l'acrylophénone est la 2-(1-morpholinométhyl)-2,3,4-triméthoxyacrylophénone.

13. Procédé suivant l'une ou l'autre des revendications 1 et 2, caractérisé en ce que l'acrylophénone est la 2-(4-méthyl-1-pipérazinylméthyl)acrylophénone.

14. Procédé suivant la revendication 2, caractérisé en ce que l'acrylophénone est le chlorhydrate de 6-(3-diméthylamino-2-méthylène-propionyl)-3-méthyl-benzoxazolinone.

15. Procédé suivant la revendication 2, caractérisé en ce que l'acrylophénone est le chlorhydrate de 6-(3-diéthylamino-2-méthylène-propionyl)-3-méthyl-benzoxazolinone.

16. Procédé suivant la revendication 2, caractérisé en ce que l'acrylophénone est le chlorhydrate de 6-(3-morpholino-2-méthylène-propionyl)-3-méthyl-benzoxazolinone.

17. Procédé suivant l'une ou l'autre des revendications 1 et 2, caractérisé en ce que l'acrylophénone est le chlorhydrate de 2-(1-morpholinométhyl)-3,4-dichloroacrylophénone.

18. Procédé suivant l'une ou l'autre des revendications 1 et 2, caractérisé en ce que l'acrylophénone est le chlorhydrate de 2-(1-morpholinométhyl)-2,4-dichloroacrylophénone.

19. Procédé suivant l'une ou l'autre des revendications 1 et 2, caractérisé en ce que l'acrylophénone est le chlorhydrate de 2-(1-morpholinométhyl)-p-nitroacrylophénone.

0156785

Office européen des brevets

# RAPPORT DE RECHERCHE EUROPEENNE

Numéro de la demande

EP 85 87 0036

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| X | CHEMICAL ABSTRACTS, volume 99, no. 9, 29 août 1983, page 413, abrégé 68107q, Columbus, Ohio, US; R.B. GWATKIN et al.: "Effect of compounds structurally related to taurine and of taurine uptake inhibitors on the motility of hamster sperm in vitro", & Gamete Res. 1983, 7(4), 347-50 * Abrégé * --- | 1,21 | C 07 C 97/10<br>C 07 D 295/10<br>C 07 D 263/56<br>A 61 K 31/135<br>A 61 K 31/395 |
| X | CHEMICAL ABSTRACTS, volume 95, no. 13, 28 septembre 1981, page 649, abrégé 114971j. Columbus, Ohio, US; R.C. GUPTA et al.: "N-substituted alpha-aminoalkylacrylophenones and sme related compounds: a new class of spermicidal agents", & Indian J. Chem. Sect. B 1981, 20B(4), 303-7 * Abrégé * --- | 1-21 | DOMAINES TECHNIQUES RECHERCHES (Int. Cl.4)<br>C 07 C 97/00<br>C 07 D 295/00<br>C 07 D 263/00<br>A 61 K 31/00 |
| X | CHEMICAL ABSTRACTS, volume 101, no. 11, 10 septembre 1984, page 59, abrégé 83944p, Columbus, Ohio, US; L. LESIEUR et al.: "Antimicrotubular activity of 2-(4-methyl-1-piperazinylmethyl)acrylophenone dihydrochloride", & Acta Ther. 1984, 10(2), 145-52 --- -/- | 1,12, 19-21 | |

Le présent rapport de recherche a été établi pour toutes les revendications

| Lieu de la recherche | Date d'achèvement de la recherche | Examinateur |
|---|---|---|
| LA HAYE | 11-06-1985 | MOREAU J.M. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503 03 82

0156785

Numero de la demande

Office européen des brevets

# RAPPORT DE RECHERCHE EUROPEENNE

EP 85 87 0036

Page 2

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl 4) |
|---|---|---|---|
| X | JOURNAL OF PHARMACEUTICAL SCIENCES, volume 72, no. 8, août 1983, pages 887-891, J.R. DIMMOCK et al.: "Evaluation of sme mannich bases derived from substituted acetophenones against P-388 lymphocytic leukemia and on respiration in isolated rat liver mitochondria", * Tabelle II, page 889, VII * --- | 1,9,21 | |
| X | TETRAHEDRON LETTERS, volume 23, no. 5, 1982, pages 547-550, Pergamon Press Ltd. (GB); A. HOSOMI et al.: "A novel aminomethylation of silyl enol ethers with aminomethyl ethers catalyzed by iodotrimethylsilane or trimethylsilyl trifluoromethanesulfonate", * Page 548, no. 9 * --- | 1 | DOMAINES TECHNIQUES RECHERCHES (Int. Cl.4) |
| X | JOURNAL OF ORGANIC CHEMISTRY, volume 45, no. 24, 21 novembre 1980, American Chemical Society, pages 4983-4984, N. CROMWELL et al.: "Mobile keto allyl systems. 18. Synthesis and chemistry of N-substituted and N,N-disbstituted 2-benzoyl-1-amino-3-propenes" * Tabelle I * --- -/- | 1,2 | |

Le présent rapport de recherche a été etabli pour toutes les revendications

| Lieu de la recherche | Date d'achèvement de la recherche | Examinateur |
|---|---|---|
| LA HAYE | 11-06-1985 | MOREAU J.M. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503 03 82

0156785

Office européen des brevets

# RAPPORT DE RECHERCHE EUROPEENNE

Numéro de la demande

EP 85 87 0036



## DOCUMENTS CONSIDERES COMME PERTINENTS

| Catégorie | Citation du document avec indication, en cas de besoin, des parties pertinentes | Revendication concernee | CLASSEMENT DE LA DEMANDE (Int Cl.4) |
|---|---|---|---|
| X | EUROPEAN JOURNAL OF MEDICINAL CHEMISTRY, volume 11, no. 4, juillet-août 1976, pages 343-350; H. ULBRICH et al.: "Substituierte Perhydro-pyrido (1,2-a)(1.4)diazepine und Perhydro-pyrido(2.1-c)(1.4)oxazepine", * Page 344, no. 13 * --- | 1 | |
| X | CHEMICAL ABSTRACTS, volume 73, no. 9, 31 août 1970, page 283, abrégé 45071x, Columbus, Ohio, US; W. BACK: "Synthesis of O-(acylamino)-beta-(dimethylamino)propiophenones. 1. Behavior of O-nitro- and O-(ethoxycarbonylamino)acetophenone in the Mannich reaction", & Arch. Pharm. (Weinheim) 1970, 303(6), 465-70 * Abrégé * --- | 1 | DOMAINES TECHNIQUES RECHERCHES (Int. Cl.4) |
| X | CHEMICAL ABSTRACTS, volume 75. no. 3, 19 juillet 1971, page 416, abrégé 19857e, Columbus, Ohio, US; W. BACK: "Synthesis of O-acrylamino-beta-(dimethylamino)propiophenones. 3. Aminomethylation of 2-nitro-5-methoxy- and 2-nitro-5-benzyloxyacetophenone", & Arch. Pharm. (Weinheim) 1971, 304(1), 27-31, * Abrégé * --- -/- | 1 | |

Le présent rapport de recherche a eté établi pour toutes les revendications

| Lieu de la recherche | Date d'achèvement de la recherche | Examinateur |
|---|---|---|
| LA HAYE | 11-06-1985 | MOREAU J.M. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

0156785

Office européen des brevets

# RAPPORT DE RECHERCHE EUROPEENNE

Numéro de la demande

EP 85 87 0036



## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| X | CHEMICAL ABSTRACTS, volume 84, no. 9, 1 mars 1976, page 464, abrégé 58837d, Columbus, Ohio, US; P. MESSINGER et al.: "Sulfones as chemical transports for germicidal compounds. 6. Preparation of sulfonyl ketone Mannich bases", & Arch. Pharm. (Weinheim, Ger.) 1975, 308(10), 737-42 * Abrégé * --- | 1,2 | |
| P,X | CHEMICAL ABSTRACTS, volume 101, no. 13, 24 septembre 1984, page 46, abrégé 103881w, Columbus, Ohio, US; C. BRUNET et al.: "Preliminary note: effect of microtubule inhibitors with acrylophenone structure on Triton WR 1339 induced hyperlipidemia in rats", & Methods Find. Exp. Clin. Pharmacol. 1984, 6(5), 227-30 * Abrégé * ----- | 1-21 | |

DOMAINES TECHNIQUES RECHERCHES (Int. Cl.4)

Le présent rapport de recherche a été établi pour toutes les revendications

| Lieu de la recherche | Date d'achèvement de la recherche | Examinateur |
|---|---|---|
| LA HAYE | 11-06-1985 | MOREAU J.M. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire
T : théorie ou principe à la base de l'invention
E : document de brevet antérieur, mais publié à la date de dépôt ou après cette date
D : cité dans la demande
L : cité pour d'autres raisons
& : membre de la même famille, document correspondant

OEB Form 1503 03 82